# EUROPEAN PATENT SPECIFICATION

(11) **EP 2 344 512 B1**
(45) Date of publication and mention of the grant of the patent: **19.12.2012**
(21) Application number: 09737474.8
(22) Date of filing: 16.09.2009
(51) Int. Cl.: C07F 15/00, A61K 31/28

(54) **PLATINUM COMPLEX WITH ANTITUMOR ACTIVITY**
PLATINKOMPLEX MIT ANTITUMORWIRKUNG
COMPLEXE DU PLATINE PRÉSENTANT UNE ACTIVITÉ ANTITUMORALE

(30) Priority: 18.09.2008 IT MI20081660
(43) Date of publication of application: 20.07.2011
(73) Proprietor: Cell Therapeutics, Inc., Seattle, WA 98119 (US)
(72) Inventor: DE MUNARI, Sergio, SEATTLE WA 98119 (US); DI GIOVINE, Stefano, SEATTLE WA 98119 (US); GRUGNI, Mario, SEATTLE WA 98119 (US); NICOLI, Paola, SEATTLE WA 98119 (US); PAGANELLI, Alessandro, SEATTLE WA 98119 (US); PARDI, Gianluca, SEATTLE WA 98119 (US); PEZZONI, Gabriella, SEATTLE WA 98119 (US)
(74) Representative: Minoja, Fabrizio
(86) International application number: PCT/IB2009/006974
(87) International publication number: WO 2010/032131

(56) References cited:
- WO-A-2007/071415

## Description

### Field of the invention

Object of the present invention is a platinum complex endowed with antitumor activity, a process for its preparation, pharmaceutical compositions containing it and its use for the preparation of a medicament useful for the treatment of tumor pathologies.

### Background of the invention

Platinum complexes are among the chemotherapeutic drugs more effective for the treatment of solid tumors. In particular, cisplatin [cis-diclorodiammineplatinum(II); CDDP] is one of the more frequently used and effective antitumor agents, although its administration is limited by some serious side effects. Moreover, tumor cells resistant to this medicament frequently develop and the majority of solid tumors (for example, tumors of the lung, of the colon-rectum and gastric tumors) do not respond to cisplatin or to other chemotherapeutic agents. (E. Wong, C.M. Giandomenico, Current Status of Platinum-Based Antitumor Drugs, Chem. Rev. 1999, 99, 2451-2466).

In order to identify novel platinum complexes endowed with reduced toxicity, a wider spectrum of antitumor activity and devoid of cross resistance with cisplatin, a large number of cisplatin analogues were studied in the last decades. These efforts resulted in second generation platinum complexes which have shown a profile of antitumor activity similar to that of cisplatin in clinical studies. Among these complexes, carboplatin, the second platinum complex to reach the commercialization phase, is devoid of the mayor limitations of toxicity of cisplatin, but it has a similar spectrum of antitumor activity. A third generation platinum complex recently introduced in the clinical practice is oxaliplatin. Other complexes under testing are the compound AMD0473 and satraplatin. However, none of these novel analogues seems to be capable of overcoming the fundamental problems associated with the therapy with cisplatin, i.e. the enlargement of the panel of tumors responding to the treatment and the overcoming of resistance.

Finally, another problem associated with the platinum complexes currently used in therapy is that, after administration by the intravenous route, they are prone to bind irreversibly to plasma proteins through a covalent bond, The kinetics of this process depends on the contact time, with more than 90% of the medicament being bound within few hours from administration. The high irreversible binding to plasma proteins may impair the effectiveness of the compounds in humans. (S.S. Jacobs et al., Clinical Cancer Research, Vol. 11, 1669-1674, 2005 and references cited therein).

Lipid soluble platinum complexes related to cisplatin are described in US 5,117,022 and in US 6,613,799. The compounds described in US 5,117,022 are useful for incorporation into liposomes, whereas for the complexes described in US 6,613,799 a high specificity and selectivity for the tumor cells is reported when the compounds are administered in a contrast medium such lipiodol.

Bisplatinum complexes characterized by the presence of a diammine or polyamine ligand which bridges the two platinum atoms and useful for the treatment of tumor pathologies are described in US 4,797,393, US 5,107,007, US 6,022,892 and US 6,596,889. In particular, US 6,022,892 and US 6,596,889 disclose bisplatinum complexes characterized by the presence of un polyamine ligand. These compounds have a potent cytotoxic activity against murine and human tumor cell lines resistant to cisplatin, such as the L1210/CDDP murine leukemia and the A2780/CDDP human ovary carcinoma cell lines. In vivo activity in an experimental tumor model resistant to cisplatin is also reported for the compounds. The international application WO2007/071415 describes bisplatinum complexes characterized by the presence of carboxylato ligands in the platinum coordination sphere, which are endowed with reduced binding to plasma proteins, show a better stability in plasma towards the processes of de-platination and are capable of inhibiting tumor growth in various experimental models. It has now been found that a compound not specifically described in US 6,022,892 is particularly advantageous in terms of stability and toxicity.

### Disclosure of the invention

The present invention relates to {µ-(1,8,11,18-Tetraazaoctadecane-N1,N18)bis[trans-diammine(butyrato-O)platinum(II)]} tetranitrate, of formula (I): 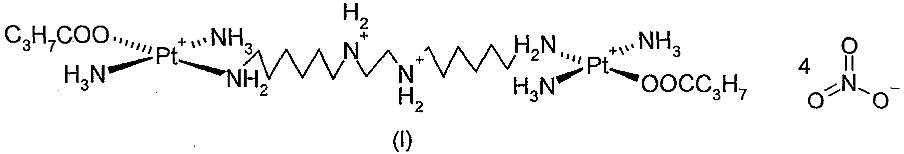 and to its use for the preparation of pharmaceutical compositions for the therapy of tumors, in particular for the preparation of medicaments for the treatment of mammals affected by tumors which can be treated or are resistant to cisplatin.

The compound can be prepared by reaction of {µ-(1,8,11,18-tetraazaoctadecane-N1,N18)bis[trans-diammine(dichloro)platinum(II)]} tetranitrate with silver butyrate, according to the method described in US 6,022,892 and illustrated in greater detail in the example below.

The pharmaceutical compositions object of the invention contain a therapeutically effective amount of the compound of formula (I) in admixture with conventional carriers and excipients, described, for example in Handbook of Pharmaceutical Excipients, Pharmaceutical Press, V edition.

The effective dose of the compound (I) can be determined by the expert clinician according to conventional methods. The relationship between the dosages used for animals of various species and sizes and those for humans (on the basis of mg/m² of body area) is described by Freirech et al., Quantitative Comparison of Toxicity of Anticancer Agents in Mouse, Rat, Hamster, Dog, Monkey and Man, Cancer Chemother. Rep., 50, N.4, 219-244 (1986). However, the patient will typically receive doses of the complex which range from 0.05 to 200 mg/kg of body weight, with a dosage regimen which will vary depending on a number of factors well known to the expert clinician.

The treatment regimen can conveniently be varied, as well known to the expert clinician, depending on the type of tumor to be treated and on the conditions of the patient.

It will be possible to administer the compound of the invention though the parenteral or oral route.

The pharmaceutical compositions for parenteral use comprise sterile saline solutions, as defined above, or sterile powders for the extemporaneous preparation of the solutions, as well as oily preparations for the intramuscular (im) or intraperitoneal (ip) administration.

The compound of the invention can be administered preferably as aqueous sterile solution, optionally containing sodium chloride in appropriate concentration (0.1 - 0.9 mg/ml). The solutions are administered preferably through the intravenous (iv) o intra-arterial (ia) route, although in particular cases other administration forms can be used.

Pharmaceutical compositions useful for the oral administration comprise, for example, syrups or similar liquid forms, as well as solid forms such as tablets, capsules and the like.

The pharmaceutical compositions according to the present invention are prepared following conventional methods, such as those reported in Remington's Pharmaceutical Sciences Handbook, XVII Ed., Mack Pub., N.Y., U.S.A.

In some instances it can be advantageous to administer the platinum complex of formula (I) together with one or more agents which enhance the antitumor activity or which alleviate the unwanted side effects which can be associated with the therapy with platinum complexes; for example, the platinum complex of the invention can be administered together with reduced glutathione, as described in GB 2,174,905 and in U.S. 4,871,528.

Moreover, it can be advantageous to administer the platinum complex of the present invention in combination with other antitumor platinum complexes. Therefore, a further object of the present invention are pharmaceutical compositions containing in combination with another platinum complex having antitumor activity and conventional excipients and/or carriers.

### EXAMPLE

### Preparation 1 - Silver butyrate

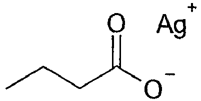

The reaction was carried out in milliQ H₂O using actinic glassware. To a solution of sodium butyrate (0.5 g, 4.5 mmol in H₂O (10 mL) was added a solution of AgNO₃ (0.771 g, 4.5 mmol) in H₂O(5 mL) drop by drop and under stirring. The precipitation of a solid occurred immediately and the resulting suspension was kept under stirring for 30 minutes. The solid was collected, washed with H₂O(20 mL) and dried under vacuum at 35°C to give 0.632 g (yield 71 %) of product.

| | | | |
|---|---|---|---|
| Elemental | Calculated | C 24.64% | H 3.62% |
| analysis | Found | C 24.62% | H 3.60% |

¹H NMR (DMSO-d₆): δ 2.07 (2H, t, J=7.28 Hz); 1.51 (2H, dt, J=7.29, 7.31); 0.87 (3H, t, J=7.36).

### Preparation 2 - {µ-(1,8,11,18-Tetraazaoctadecane-N1,N18)bis[trans-diammine(butyrato-O)platinum(II)]} tetra nitrate

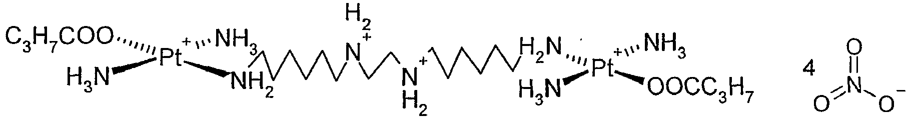

The reactions were carried out with shielding from light and in milliQ H₂O. To a solution of {µ-(1,8,11,18-tetraazaoctadecane-N1,N18)bis[trans-diammine(dichloro)platinum(II)]} tetranitrate (0.4 g, 0.385 mmol) in H₂O (36 mL) silver butyrate (0.3 g, 1.539 mmol) was added and the resulting suspension was left under stirring at room temperature for 48 hours. The solid was removed filtering twice the mixture over a double microfiber-filter; the filter was washed with 2 ml of H₂O which were combined with the filtrate. The filtrate was extracted with a solution of dithizone (0.2 g, 0.780 mmol) in CHCl₃ (38 mL) and washed with CHCl₃ (5 x 19 mL). The aqueous phase was separated from the organic one and evaporated to dryness (35°C) under reduced pressure; the solid residue was collected and dried under vacuum at 35°C. A suspension of the solid in CHCl₃ (60 mL) was kept under stirring for 1 hour; the solid was collected, washed with CHCl₃ and hexane and dried under vacuum at 35°C to give 0.348 g (yield 79%) of product.

m.p. 128.2-134.4°C

| Elemental analysis | | | | |
|---|---|---|---|---|
| Calculated | C 23.16% | H 5.48% | N 14.73% | Pt 34.19% |
| Found | C 21.95% | H 5.17% | N 13.93% | Pt 32.84% |

MS: 1103.0, [MH+C₃F₇COOH-4HNO₃]⁺

¹H NMR (D₂O): δ 3.93 (2H, s); 3.33 (4H, s); 3.05 (4H, t, J=7.68 Hz); 2.62 (4H, m); 2.24 (4H, t, J=7.40); 1.68 (8H, m); 1.53 (4H, m); 1.40 (8H, m); 0.87 (6H, t, J=7.41).

¹⁹⁵Pt NMR (D₂O): δ -2167.70.

## Claims

1. The compound of formula (I):

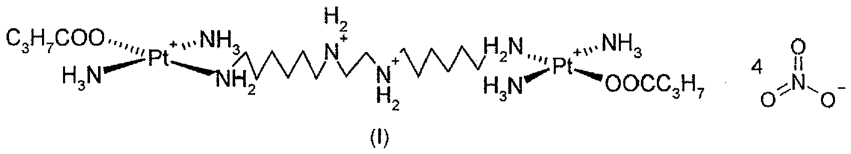

2. The compound of formula (I) according to claim 1 as medicament.

3. Pharmaceutical compositions containing the compound of formula (I) according to claim 1 in admixture with pharmaceutically acceptable carriers and/or excipients.

4. Use of the compound of formula (I) as defined in claim 1 for the preparation of a medicament for the treatment of tumors.

5. The use according to claim 4 wherein the tumor is resistant to therapy with cisplatin.

## Patentansprüche

1. Verbindung der Formel (I):

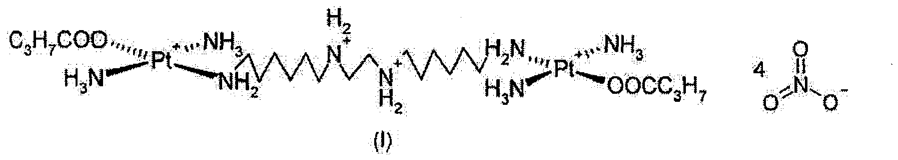

2. Verbindung der Formel (I) gemäß Anspruch 1 als Medikament.

3. Pharmazeutische Zusammensetzungen, enthaltend die Verbindung der Formel (I) gemäß Anspruch 1 in Mischung mit pharmazeutisch verträglichen Trägern und/oder Exzipienten.

4. Verwendung der Verbindung der Formel (I) wie in Anspruch 1 definiert, für die Herstellung eines Medikaments für die Behandlung von Tumoren.

5. Verwendung gemäß Anspruch 4, wobei der Tumor gegen eine Therapie mit Cisplatin resistent ist.

## Revendications

1. Composé de formule (I) :

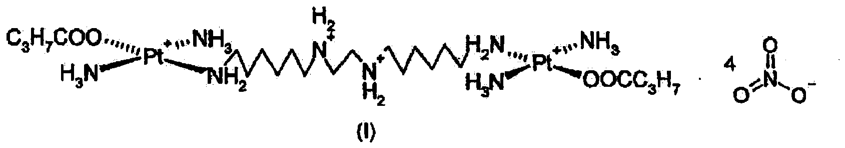

2. Composé de formule (I) selon la revendication 1 comme médicament.

3. Compositions pharmaceutiques contenant le composé de formule (I) selon la revendication 1 en mélange avec des vecteurs et/ou excipients pharmaceutiquement acceptables.

4. Utilisation du composé de formule (I) tel que défini dans la revendication 1 pour la préparation d'un médicament pour le traitement des tumeurs.

5. Utilisation selon la revendication 4 où la tumeur est résistante à une thérapie avec le cisplatine.
